# FASCICULE DE BREVET EUROPEEN

(11) **EP 3 387 000 B1**
(45) Date de publication et mention de la délivrance du brevet: **14.08.2019**
(21) Numéro de dépôt: 16825461.3
(22) Date de dépôt: 09.12.2016
(51) Int. Cl.: C07H 7/033, C07H 15/04, A01N 25/30, A01P 3/00, A01P 1/00

(54) **PROCÉDÉ D'OBTENTION DE COMPOSITIONS TENSIOACTIVES À BASE DE L-GULURONAMIDES D'ALKYLE**
VERFAHREN ZUR GEWINNUNG VON AUS ALKYL-L-GULURONAMIDEN HERGESTELLTEN TENSIDZUSAMMENSETZUNGEN
METHOD FOR OBTAINING SURFACTANT COMPOSITIONS MADE FROM ALKYL-L-GULURONAMIDES

(30) Priorité: 11.12.2015 FR 1562228
(43) Date de publication de la demande: 17.10.2018
(73) Titulaire: École Nationale Supérieure De Chimie, 35000 Rennes (FR)
(72) Inventeur: BENVEGNU, Thierry, 35000 Rennes (FR); SARI-CHMAYSSEM, Nouha, 35700 Rennes (FR); TAHA, Samir, Beyrouth 99999 (LB); MAWLAWI, Hiba, Tripoli 99999 (LB)
(74) Mandataire: Novagraaf Technologies
(86) Numéro de dépôt international: PCT/FR2016/053290
(87) Numéro de publication internationale: WO 2017/098174

(56) Documents cités:
- WO-A2-03/104248
- US-A- 5 412 118
- N BOLLENBACK J W LONG ET AL: "BURCKHARDT HELFEKICH und ADELHEID BERCER UBER DIE SYNTHESE VON GLUCURONIDEN')", CHEMISCHE BERICHTE, vol. 90, no. 11, 30 novembre 1957 (1957-11-30), pages 2492-2498, XP055263352, DE ISSN: 0009-2940
- MARY FIESER ET AL: "Synthetic Emulsifying Agents", JOURNAL OF THE AMERICAN CHEMICAL SOCIETY, AMERICAN CHEMICAL SOCIETY, US, vol. 78, no. 12, 20 juin 1956 (1956-06-20) , pages 2825-2832, XP002631089, ISSN: 0002-7863, DOI: 10.1021/JA01593A049

## Description

### Domaine technique

La présente invention se rapporte à un nouveau procédé direct d'obtention de compositions tensioactives comprenant des L-guluronamides d'alkyle ou de mélanges de L-guluronamides et de D-mannuronamides d'alkyle à partir de matières premières biosourcées (alginates, oligoalginates, poly(oligo)guluronates, algues brunes) ou biocompatibles/biodégradables.

La présente invention trouve par exemple des applications dans les tensioactifs, notamment pour la cosmétologie, le domaine phytosanitaire, agroalimentaire et la détergence (industrielle).

Dans la description ci-dessous, les références entre crochets (**[]**) renvoient à la liste des références présentée à la fin du texte.

### État de la technique

Les tensioactifs à base de carbohydrates représentent une classe importante de composés amphiphiles dont l'intérêt croissant peut s'expliquer par des facteurs d'ordre fonctionnel, économique et environnemental (Hill et Lehen-Ferrenbach, 2009) **[1].** Les dérives amides de sucres caractérisés par la présence d'une fonction amide reliant la tête sucre hydrophile à la chaîne lipophile présentent l'avantage d'être résistants vis-à-vis de l'hydrolyse en milieux neutre et alcalin, notamment comparativement aux dérivés esters (Laurent et al., 2011) **[2].** Si des travaux ont déjà montré la possibilité d'accéder à des dérivés amides à partir d'acides uroniques comme l'acide glucuronique et l'acide galacturonique issus de l'hydrolyse d'hémicelluloses ou de pectines (Laurent et al., 2011, précité) **[2],** il existe peu de travaux permettant de valoriser des polysaccharides d'origine algale. Un seul exemple de tensioactif amide est issu de la transformation d'oligomères de l'acide D-mannuronique provenant de la dépolymérisation d'alginates. Par contre, la préparation de compositions tensioactives sous forme amide à base d'acide L-guluronique ou de mélanges d'acide L-guluronique et d'acide D-mannuronique et permettant de valoriser la totalité des saccharides présents dans le biopolymère n'a pas été développée à ce jour.

Il existe trois classes distinctes de tensioactifs à base de saccharides : les esters (esters de sorbitan, sucroesters), les acétals (alkylpolyglucosides) et les amides (alkyl glucamides). Industriellement, les alkyl sucroamides sont produits en deux étapes : amination réductrice d'un carbohydrate avec une alkylamine, suivie de l'acylation du *N*-glycoside résultant (Demande internationale WO 9206984 ; Demande internationale WO 9303004 ; Brevet US 7,655,611; Brevet US 5,872,111) **[3-6].** De façon similaire, les gluconamides sont obtenus en deux étapes : l'oxydation d'un carbohydrate conduisant à une lactone ou un acide aldonique suivie d'une réaction avec des alkyl amines pour former des gluconamides (Brevet US 2,670,345) **[7].** Des dérivés comportant une liaison amide entre les parties hydrophile et lipophile via une liaison *N*-glycoside ont été plus récemment développés (Brevet US 7,655,611, précité) **[5].** Une autre stratégie a reposé sur la formation de tensioactifs *N*-alkylamides à partir d'acides uroniques comme l'acide glucuronique et l'acide galacturonique issus de l'hydrolyse d'hémicelluloses ou de pectines (Laurent et al., 2011, précité) **[2].** Tous ces procédés de synthèse des tensioactifs utilisent des monosaccharides comme matières premières et les conditions de synthèse sont généralement peu respectueuses de l'environnement (réactifs toxiques et non biodégradables).

Des tensioactifs mannuronamides ont été produits à partir d'oligomères de l'acide D-mannuronique (Benvegnu et Sassi, Topics in Current Chemistry, 294: 143-164, 2010; Demande internationale WO 03/104248) **[8, 9].** Le procédé repose sur la production d'oligomannuronates saturés (dépolymérisation acide) qui sont ensuite transformés en intermédiaire monosaccharide comportant deux chaînes butyle. Ce synthon est ensuite soumis à une réaction d'aminolyse à l'aide d'una amine grasse dans un solvant tel que le méthanol ou le l'isopropanol en présence ou non d'une base organique. Le dérivé *N*-acylé ainsi obtenu présente des propriétés émulsionnantes. Toutefois l'utilisation des poly(oligo)mères à base d'acide L-guluronique provenant de la dépolymérisation d'alginates (Demande internationale WO 03/099870) **[10]** ou encore de l'alginate entier n'a pas été valorisée à ce jour.

Il existe donc un réel besoin d'un nouveau procédé de synthèse de composés et compositions palliant les défauts, inconvénients et obstacles de l'art antérieur, en particulier d'un procédé permettant de maîtriser l'obtention à l'échelle industrielle, de réduire les coûts et d'améliorer les propriétés attendues des composés et compositions notamment dans le domaine des tensioactifs, et répondant au principe de la « chimie bleue ».

### Description de l'invention

Les Inventeurs ont mis au point un nouveau procédé, sans solvant, utilisant des réactifs biocompatibles / biodégradables, pour accéder simplement à des compositions tensioactives à base de L-guluronamide ou de mélanges de L-guluronamides et de D-mannuronamides directement (procédé « one-pot ») à partir de poly(oligo)guluronates, d'alginates bactériens, d'alginates raffinés et semi-raffinés (mélange d'alginate, de cellulose, d'hémicellulose et de fucane) ou d'algues brunes, évitant ainsi une étape de dépolymérisation préalable. Les poly(oligo)guluronates proviennent de la dépolymérisation d'alginates, par exemple selon le procédé décrit dans la Demande internationale WO 03/099870 **[10].** Les alginates (semi-raffinés, raffinés) et les oligoalginates sont obtenus par des traitements simples en milieux aqueux acide, à partir d'algues fraiches ou sèches, par exemple obtenus selon le protocole décrit dans l'exemple 2 ci-après selon le procédé de la Demande internationale WO 98/40511 **[12].**

Les alginates bactériens sont obtenus par exemple à partir de culture de bactéries mucoïdes (*e.g*. cf. Demande internationale WO 2009/134368) **[11]**

La présente invention a donc pour objet un procédé d'obtention d'une composition comprenant :
(i) des L-guluronamides d'alkyle de formules (la) et (Ib) : 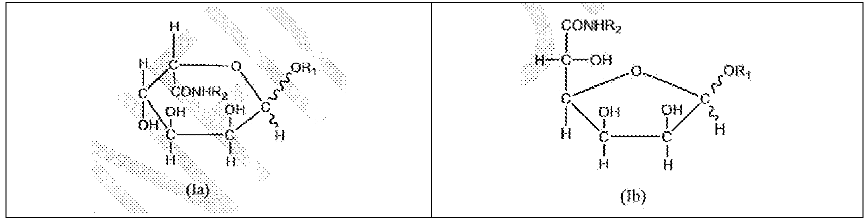 ou
(ii) un mélange de L-guluronamides d'alkyle de formules (la) et (Ib) et de D-mannuronamides d'alkyle de formules (IIa) et (IIb) : 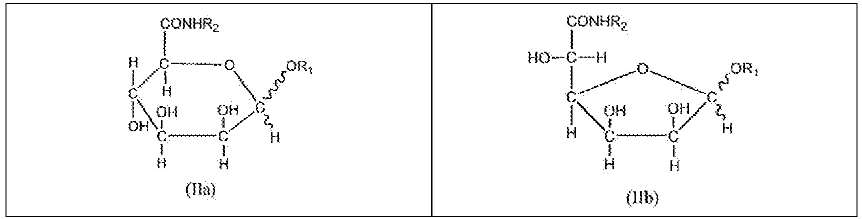 où
   - R₁ est une chaîne alkyle de 2 à 22, de préférence de 2 à 8, préférentiellement de 2 à 4, atomes de carbone, linéaire ou ramifiée, saturée ou insaturée ;
   - R₂ est un atome d'hydrogène, une chaîne alkyle de 2 à 22, de préférence de 8 à 18, préférentiellement de 12 à 18, atomes de carbone, linéaire ou ramifiée, saturée ou insaturée pouvant comporter une fonction amine terminale ;
   et caractérisé en ce que ledit procédé comprend :
   a) une étape de réaction de butanolyse et de glycosylation de Fischer à partir de poly(oligo)guluronates, d'alginates, d'oligoalginates, et/ou d'algues brunes ;
   b) une étape de réaction d'aminolyse sur le milieu réactionnel issu de l'étape a), en présence d'une amine de formule R'NH₂ où R' est composé de 2 à 22, de préférence de 8 à 18, préférentiellement de 12 à 18, atomes de carbone, linéaire ou ramifiée, saturée ou insaturée.

Par « poly(oligo)guluronates » au sens de la présente invention, on entend des blocs homopolymériques d'acide α-L-guluronique en partie sous forme de sel de sodium issus de la dépolymérisation d'alginates, par exemple selon le procédé de la Demande Internationale WO 03/099870 [10].

Par « oligoalginates » au sens de la présente invention, on entend les produits issus d'un traitement par voie enzymatique et/ou acide d'alginate, par exemple obtenus selon le protocole décrit dans l'exemple 2 ci-après selon le procédé de la Demande Internationale WO 98/40511 **[12].**

Par « alginates » au sens de la présente invention, on entend des alginates raffinés et/ou semi-raffinés, obtenus par exemple selon le protocole décrit dans l'exemple 2 ci-après. On entend également des alginates bactériens, obtenus par exemple à partir de culture de bactéries mucoïdes (*e.g.* cf. Demande internationale WO 2009/134368) **[11].**

Par « algues brunes » au sens de la présente invention, on entend les algues nommées *Phaeophyceae* ou Phéophycées dont il existe 1500 espèces (*e.g. Ascophyllum nodosum, Fucus serratus, Laminaria hyperborea, Laminaria digitata, Ecklonia maxima, Macrocystis pyrifera, Sargassum vulgare, etc...),* et dont les parois sont essentiellement composées de fucanes sulfatés et d'alginate.

Selon un mode de réalisation particulier de la présente invention, ledit procédé comprend, avant l'étape a), les étapes de préparation des alginates (semi-)raffinés, oligoalginates et poly(oligo)guluronates. Les poly(oligo)guluronates proviennent de la dépolymérisation d'alginates. Les alginates semi-raffinés proviennent de la lixiviation acide d'algues brunes suivie d'une solubilisation des alginates de sodium par augmentation du pH puis d'une séparation solide/liquide afin d'éliminer les résidus algaux. Les alginates raffinés proviennent d'une étape supplémentaire de dépigmentation par le formol et d'une étape de purification. Les oligoalginates proviennent du traitement par voie enzymatique et/ou acide de solution d'alginate. Les alginates bactériens sont obtenus par exemple à partir de culture de bactéries mucoïdes (*e.g*. cf. Demande internationale WO 2009/134368) **[11].**

Selon un mode de réalisation particulier de la présente invention, ledit procédé peut comprendre en outre une étape a') de neutralisation du milieu réactionnel issu de l'étape a), et réalisée avant l'étape b), conduisant à une composition finale incluant une quantité variable de sel d'amine grasse résiduelle. Par exemple, l'étape de neutralisation est réalisée en présence de soude 1M, jusqu'à un pH de 7.

Selon un mode de réalisation particulier de la présente invention, l'étape de butanolyse et de glycosylation de Fisher a) est réalisée en présence (i) d'eau et/ou d'un solvant ionique et/ou d'un solvant eutectique, (ii) d'un alcool ROH, linéaire ou ramifié, saturé ou insaturé, ayant de 1 à 4 atomes de carbone, de préférence du *n*-butanol, et (iii) d'un catalyseur acide tel que par exemple l'acide chlorhydrique, l'acide sulfurique, un acide alkyl sulfurique tel que l'acide décyl ou lauryl sulfurique, un acide sulfonique tel que l'acide benzènesulfonique, l'acide paratoluène sulfonique, l'acide camphosulfonique, un acide alkylsulfonique tel que l'acide méthylsulfonique, l'acide décylsulfonique, l'acide laurylsulfonique, l'acide sulfosuccinique ou un sulfosuccinate d'alkyl tel le sulfosuccinate de décyle ou sulfosuccinate de lauryle, les acides perhalohydriques, tel que l'acide perchlorique, des métaux tels que le fer, leurs oxydes ou leurs sels, comme leurs halogénures. De préférence, il s'agit d'acide alkylsulfonique ou d'acide méthanesulfonique

Par « solvant ionique » au sens de la présente invention, on entend par exemple le chlorure de 1-butyl-3-méthylimidazolium [BMIM]Cl, le bromure de 1-butyl-3-méthylimidazolium [BMIM]Br, le méthylsulfate de tris-(2-hydroxyéthyl)méthylammonium (HEMA) et l'acétate de 1-éthyl-3-méthylimidazolium [EMIM]AcO ; ledit solvant ionique comprenant typiquement jusqu'à 10% d'eau.

Par « solvant eutectique » au sens de la présente invention, on entend des systèmes formés d'un mélange eutectique de bases ou d'acides de Lewis ou Brönsted qui peuvent contenir une variété d'espèces anioniques et/ou d'espèces cationiques. Les solvants eutectiques de première génération ont été basés sur des mélanges de sels d'ammonium quaternaire avec des donneurs de liaison d'hydrogène tels que les amines et les acides carboxyliques (*e.g.* sel d'ammonium quaternaire et (hydrate de) chlorure de métal.

Cette étape a) est réalisée, par exemple, en mettant en présence un équivalent de poly(oligo)guluronates de degré de polymérisation compris entre 2 et 100, de préférence environ 35, issus de la dépolymérisation acide (Demande internatinale WO 03/099870) **[10]** d'alginates extraits des espèces *Ascophyllum, Durvillaea, Ecklonia, Laminaria, Lessonia, Macrocystis, Sargassum* and *Turbinaria,* et de préférence des alginates riches en acide L-guluronique ; de 10 à 1000 équivalents molaires d'eau ; de 2 à 300 équivalents molaires d'alcool, tel que le *n*-butanol, sont introduits, et de préférence 150 équivalents molaires ; de 10⁻³ à 10 équivalents molaires d'un catalyseur acide, tel que l'acide chlorhydrique, l'acide sulfurique, un acide alkyl sulfurique tel que l'acide décyl ou lauryl sulfurique, un acide sulfonique tel que l'acide benzènesulfonique, l'acide paratoluène sulfonique, l'acide camphosulfonique, un acide alkylsulfonique tel que l'acide méthylsulfonique, l'acide décylsulfonique, l'acide laurylsulfonique, l'acide sulfosuccinique ou un sulfosuccinate d'alkyl tel le sulfosuccinate de décyle ou sulfosuccinate de lauryle, les acides perhalohydriques, tel que l'acide perchlorique, des métaux tels que le fer, leurs oxydes ou leurs sels, comme leurs halogénures, et de préférence de 1,1 à 10 équivalents molaires d'acide alkylsulfonique, et de préférence 2,1 équivalents molaires d'acide méthylsulfonique.

La réaction est alors conduite au reflux de l'azéotrope à pression atmosphérique (montage Dean Stark), entre 130 et 135°C dans le cas du butanol, de préférence sur 12 heures. La composition ainsi formée est constituée majoritairement de composés à deux chaînes provenant de l'alcool (de préférence du butanol) dérivés de l'acide L-guluronique.

La préparation des L-guluronamides d'alkyle où la chaîne alkyle est dérivée d'une amine grasse, se poursuit par l'étape b) d'aminolyse, après abaissement de la température (de préférence jusqu'à 60°C), en ajoutant de 1 à 25 équivalents molaires d'une amine de formule R'NH₂, linéaire ou ramifiée, saturée ou insaturée où R' est composé de 5 à 22 atomes de carbone, et de préférence de 3 équivalents molaires sont ajoutés.

La réaction est conduite à une température de préférence de 65-70 °C et sous pression réduite pour le recyclage de l'alcool précédemment cité.

La composition ainsi formée constitue un produit d'usage dérivé de l'acide L-guluronique comme émulsifiants.

Les sels et les sucres n'ayant pas réagi peuvent être éliminés de cette composition par reprise dans un solvant organique, de préférence l'éther diéthylique, puis filtrés et rincés plusieurs fois avec le solvant organique. Le filtrat contenant les L-guluronamides d'alkyle est concentré pour donner une composition enrichie en produits d'intérêt qui constitue également un produit d'usage tel qu'un agent émulsifiant présentant des propriétés antibactériennes et antifongiques aux concentrations utilisées pour la formation des émulsions.

L'étape a) du procédé de l'invention est réalisée, par exemple, en mettant en présence d'un équivalent d'alginate, telles que les alginates extraits des espèces *Ascophyllum, Durvillaea, Ecklonia, Laminaria, Lessonia, Macrocystis, Sargassum* and *Turbinaria,* les oligoalginates issus d'une dépolymérisation, et de préférence des alginates riches en acide L-guluronique ; de 10 à 1000 équivalents molaires d'eau; de 2 à 300 équivalents molaires d'alcool tel que le *n*-butanol sont introduits et de préférence 150 équivalents molaires; de 10⁻³ à 10 équivalents molaires d'un catalyseur acide, tel que l'acide chlorhydrique, l'acide sulfurique, un acide alkyl sulfurique tel que l'acide décyl ou lauryl sulfurique, un acide sulfonique tel que l'acide benzènesulfonique, l'acide paratoluène sulfonique, l'acide camphosulfonique, un acide alkylsulfonique tel que l'acide méthylsulfonique (ou méthanesulfonique), l'acide décylsulfonique, l'acide laurylsulfonique, l'acide sulfosuccinique ou un sulfosuccinate d'alkyl tel le sulfosuccinate de décyle ou sulfosuccinate de lauryle, les acides perhalohydriques, tel que l'acide perchlorique, des métaux tels que le fer, leurs oxydes ou leurs sels, comme leurs halogénures, et de préférence de 1,1 à 10 équivalents molaires d'acide alkylsulfonique, et de préférence 2,5 équivalents molaires d'acide méthylsulfonique.

La réaction est alors conduite au reflux de l'azéotrope à pression atmosphérique (montage Dean Stark), entre 130 et 135°C dans le cas du butanol, de préférence sur 24 heures.

La composition ainsi formée est constituée majoritairement de composés à deux chaînes provenant de l'alcool (de préférence du butanol) dérivés de l'acide L-guluronique et de l'acide D-mannuronique.

La préparation des L-guluronamides d'alkyle et des D-mannuronamides d'alkyle où la chaîne alkyle est dérivée d'une amine grasse, se poursuit par l'étape b) d'aminolyse, après abaissement de la température (de préférence jusqu'à 60°C) selon deux protocoles possibles :
1) La réaction d'aminolyse se fait sans neutralisation préalable du milieu : en présence de 1 à 25 équivalents molaires d'une amine de formule R'NH₂, linéaire ou ramifiée, saturée ou insaturée où R' est composé de 2 à 22 atomes de carbone, et de préférence de 3 équivalents molaires. Par exemple, l'amine grasse est choisi dans le groupe constitué de la dodécylamine et de l'amine oléïque. La réaction est conduite à une température de préférence de 65-70 °C et sous pression réduite pour le recyclage de l'alcool précédemment cité. La composition ainsi formée constitue un produit d'usage dérivé de l'acide L-guluronique et de l'acide D-mannuronique comme émulsifiants. Les sels et les sucres n'ayant pas réagi peuvent être éliminés de cette composition par reprise dans un solvant organique, de préférence l'éther diéthylique, puis filtrés et rincés plusieurs fois avec le solvant organique. Le filtrat contenant les L-guluronamides d'alkyle et les D-mannuronamides d'alkyle est concentré pour donner une composition enrichie en produits d'intérêt qui constitue également un produit d'usage tel qu'un agent émulsifiant présentant des propriétés antibactériennes et antifongiques aux concentrations utilisées pour la formation des émulsions.
2) La réaction d'aminolyse se fait après neutralisation préalable du milieu : par ajout d'une solution de NaOH 1N jusqu'à un pH proche de 7. Le milieu est ensuite concentré 6 fois sous pression réduite sans aller à sec. Puis de 1 à 10 équivalents molaires d'une amine de formule R'NH₂, linéaire ou ramifiée, saturée ou insaturée où R' est composé de 5 à 22 atomes de carbone, et de préférence de 1 équivalent molaire, sont ajoutés. Par exemple, l'amine grasse est choisi dans le groupe constitué de la dodécylamine et de l'amine oléïque. La réaction est conduite à une température de préférence de 65-70°C et sous pression réduite pour le recyclage de l'alcool précédemment cité. Par la suite, de 100 à 1000 équivalents molaires d'eau, de préférence 500 équivalents sont ajoutés au milieu. Le mélange est agité environ 15 minutes à 65-70°C. Après arrêt de l'agitation, le milieu est laissé pendant environ 10 mn à cette même température de manière à ce que les produits organiques floculent. Après abaissement de la température à l'ambiante, la phase organique se solidifie et il est alors facile d'éliminer l'eau chargée des sels.

Les compositions ainsi formées par le procédé de l'invention constituent des produits d'usage dérivés de l'acide L-guluronique et de l'acide D-mannuronique tel que des agents émulsifiants présentant des propriétés antibactériennes et antifongiques aux concentrations utilisées pour la formation des émulsions.

La présente invention a également pour objet une composition obtenue par le procédé selon l'invention. Les compositions de l'invention sont constituées de dérivés d'acide L-guluronique ou des deux acides uroniques (acide L-guluronique et acide D-mannuronique) issu du même polysaccharide. En outre, les dérivés de l'acide L-guluronique et de l'acide D-mannuronique qui constituent les compositions de l'invention sont sous forme à la fois de pyranosides (cycle à 6 chaînons) et de furanosides (cycles à 5 chaînons). En fonction de la longueur de la chaîne et de la nature des chaînes d'alkyle, les compositions de l'invention seront considérées comme des agents émulsionnants pour des émulsions eau dans huile (E/H) ou huile dans eau (H/E). De plus, elles pourront présenter des propriétés antibactériennes et antifongiques.

La présente invention a donc également pour objet l'utilisation d'une composition selon l'invention comme agent tensioactif. De préférence, ledit agent tensioactif est choisi dans le groupe constitué des agents solubilisants, hydrotropes, mouillants, moussants, émulsionnants, émulsifiants et/ou détergents.

La présente invention a également pour objet une composition selon l'invention pour une utilisation comme agent antibactérien et/ou antifongique.

La présente invention a également pour objet un tensioactif comprenant une composition selon l'invention. Ledit tensioactif peut présenter les propriétés suivantes :

| Nombre d'atomes de carbone de la chaîne lipophile (alkyle R2) : | Tensioactif à deux chaînes lipophiles : D-mannuronamides d'alkyl et/ou L-guluronamides d'alkyle |
|---|---|
| Entre 1 et 6 | Agents hydrotropes et/ou solubilisants |
| Entre 6 et 14 | Agents émulsifiants huile dans eau (H/E) et/ou eau dans huile (E/H) |
| Entre 16 et 22 | Agents émulsifiants eau dans huile (E/H) |

La présente invention a également pour objet un antifongique et/ou antibactérien comprenant une composition selon l'invention.

Le procédé de l'invention conduit à des compositions tensioactives originales en utilisant exclusivement des matières premières biosourcées (poly(oligo)guluronates, alginates, oligoalginates, algues brunes) ou biocompatibles / biodégradables :
- en mettant en oeuvre une méthodologie qui permet de transformer l'acide L-guluronique provenat de poly(oligo)guluronates ou à la fois l'acide L-guluronique et l'acide D-mannuronique, *i.e.* les deux sucres uroniques constitutifs des alginates, pour conduire à des compositions tensioactives constituées exclusivement de l'acide L-guluronique ou des deux saccharides (l'acide L-guluronique et l'acide D-mannuronique) ;
- en utilisant des conditions qui peuvent permettre de s'affranchir de l'étape préalable de dépolymérisation de l'alginate et donc de l'utiliser directement ;
- en proposant des conditions qui répondent aux principes de la chimie bleue, réactions sans solvants organiques autres que les alcools/amines réactifs, ne produisant pas de déchets (recyclage des alcools à chaînes courtes (*n*-butanol, etc...)) et utilisant des réactifs biodégradables (acide méthane sulfonique et analogues) ;
- en réalisant l'ensemble des réactions selon un procédé « one pot » sans isolement, ni purification des intermédiaires réactionnels ;
- en contrôlant la quantité d'amine grasse utilisée lors de l'étape d'aminolyse et les conditions de neutralisation de l'acide utilisé lors de première phase du procédé, il est possible d'aboutir à des compositions tensioactives comprenant des proportions variables de sels d'alkyl ammonium ;
- en utilisant des conditions simples de purification partielle des bruts réactionnels et d'isolement des compositions tensioactives qui permettent d'aboutir aux composés dérivés et aux compositions à des prix plus compétitifs par rapport au marché actuel.

Le procédé de l'invention permet de produire des compositions dérivées de l'acide L-guluronique ou dérivées à la fois de l'acide L-guluronique et de l'acide D-mannuronique sous forme d'amide qui présentent l'avantage de former des émulsions eau dans huile (E/H) et huile dans eau (H/E) très stables en comparaison avec des émulsifiants commerciaux, et de posséder des propriétés antibactériennes et antifongiques aux concentrations utilisées pour la formation des émulsions.

Ainsi le procédé de l'invention permet à la fois de réduire les coûts de production des compositions tensioactives et de proposer de nouvelles compositions dans un objectif d'amélioration des performances (propriétés émulsionnantes notamment).

D'autres avantages pourront encore apparaître à l'homme du métier à la lecture des exemples ci-dessous, illustrés par les figures annexées, donnés à titre illustratif.

### Brève description des figures

- La figure 1 représente la mesure des tensions interfaciales des compositions GN12, GN18, AlgN12, AlgN18 et Algn12brut.
- La figure 2 représente la mesure du pouvoir émulsionnant des compositions GN12, GN18, AlgN12, AlgN18 et Algn12brut en comparaison de références commerciales Xylance® et Montanov®.

### EXEMPLES

### EXEMPLE 1: PROCÉDÉ D'OBTENTION DE L-GULURONAMIDES D'ALKYLE À PARTIR DE POLY(OLIGO)GULURONATES

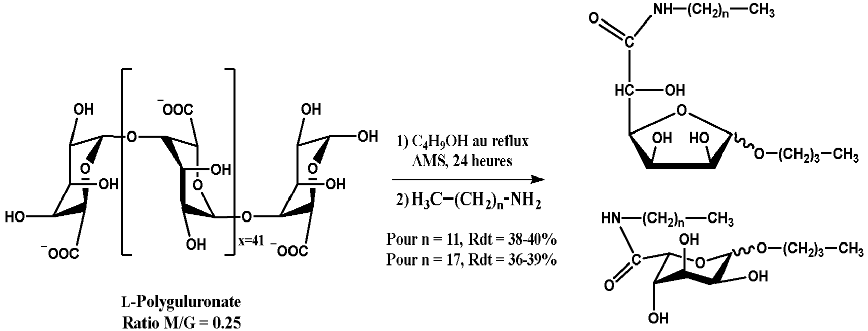

Préparation matières premières : les poly(oligo)guluronates peuvent par exemple être obtenus selon procédé de la Demande Internationale WO 03/099870 **[10].**

### 1) Réaction de butanolyse et de glycosylation de Fischer

1 g de L-poly(oligo)guluronate de sodium (7500 g/mol, Degré de polymérisation = 44) dont le ratio M/G est 0,25 (5,71 mmol, 1 éq) a été mélangé avec 2 mL d'eau distillée. 80 mL (150 éq) de butanol ont été ajoutés à la solution d'alginate sous agitation avec 778 µl d'acide méthane sulfonique (12 mmol, 2,1 éq). Le milieu a été agité à la température de reflux du butanol (130-135°C) pendant 12 heures. Les eaux présentes dans le milieu et celles formées au cours de la réaction ont été éliminées dans un Dean Stark rempli de butanol, par une distillation azéotropique eau-butanol. Etant plus dense que le butanol, l'eau se déplace vers le fond du Dean Stark et quelques ml de butanol passent dans le ballon pour conserver le volume initial. Après 12 heures, une chromatographie sur couche mince (CH₂Cl₂/CH₃OH 95/5 v/v) a été réalisée pour le milieu réactionnel pour s'assurer que le produit attendu a bien été synthétisé.

### 2) Réaction d'aminolyse

La température du milieu a été abaissée jusqu'à 60°C avant d'ajouter 3 équivalents molaires (17,14 mmol) d'amine grasse (Pour n = 11, 3,18 grammes et pour n = 17, 4,62 grammes) nécessaire pour augmenter le pH à 8,5. Après 30 minutes d'agitation à 65°C et sous pression réduite de 150 mbar, le butanol a été évaporé en diminuant la pression de 150 mbar à 6 mbar sous une période de 1 heure. Le milieu a été laissé sous pression réduite de 6 mbar pendant 1 heure et demi pour s'assurer de l'évaporation des traces de butanol qui se formaient.

Le résidu obtenu a été repris dans l'éther diéthylique puis filtré sur fritté et rincé plusieurs fois avec l'éther diéthylique pour éliminer les sels et le sucre de départ non réagi. Le filtrat (contenant nos guluronamides d'alkyle) a été concentré sous vide pour éliminer l'éther diéthylique. Une huile brune a ainsi été obtenue.

Pour n = 11, après chromatographie éventuelle de l'huile obtenue sur colonne de gel de silice (80 grammes, en utilisant l'éluant CH₂Cl₂/CH₃OH 95/5 v/v), a été déterminée la présence de 0,95 g (2,27 mmol, 40% de rendement) d'un mélange de 4 formes d'isomères de *N*-(12-dodécyl)-*n*-butyl β-L-gulurofuranosiduronamide (37%), *N*-(12-dodécyl)-*n*-butyl α-L-gulurofuranosiduronamide (21%), *N*-(12-dodécyl)-*n*-butyl α-L-guluropyranosiduronamide (28%), et *N*-(12-dodécyl)-*n*-butyl β-L-guluropyranosiduronamide (14%). Le ratio pyranose/furanose est de 0,74. **Cette composition tensioactive a été nommée GN12.**

Pour n = 17, après chromatographie éventuelle de l'huile obtenue sur colonne de gel de silice (80 grammes, en utilisant l'éluant CH₂Cl₂/CH₃OH 95/5 v/v), a été déterminée la présence de 1,08 g (2,16 mmol, 38% de rendement) d'un mélange de 4 formes d'isomères de *N*-(18-octadécyl)-*n-*butyl β-L-gulurofuranosiduronamide (36%), *N*-(18-octadécyl)-*n*-butyl α-L-gulurofuranosiduronamide (23%), *N*-(18-octadécyl)-*n*-butyl α-L-guluropyranosiduronamide (25%), et *N*-(18-octadécyl)-*n*-butyl β-L-guluropyranosiduronamide (16%). Le ratio pyranose/furanose est de 0,69. **Cette composition tensioactive a été nommée GN18.**

### EXEMPLE 2 : PROCÉDÉ D'OBTENTION DE L-GULURONAMIDES ET D-MANNURONAMIDES D'ALKYLE À PARTIR D'ALGINATES

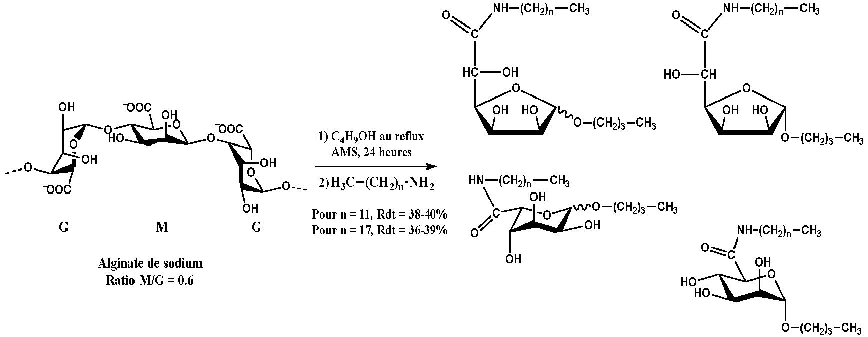

Préparation des matières premières : Les procédés d'extraction des alginates sont classiquement utilisés au CEVA (René Perez « La culture des algues marines dans le monde », Ifremer). Ils mettent en jeu une lixiviation acide d'algues fraîches ou sèches (lavage à l'eau de mer des algues récoltées, dépigmentation dans le formol, broyage, extraction avec de l'acide sulfurique à 0,2N à température ambiante, égouttage et rinçage des algues lixiviées avec de l'eau distillée), suivi d'une solubilisation des alginates de sodium par augmentation du pH du milieu puis d'une séparation solide/liquide afin d'éliminer les résidus algaux (addition d'une solution de Na₂CO₃ à 1,5% à 50g sec de matière algale lixiviée selon un ratio algue sèche/solution Na₂CO₃ à 1,5% de 0,025, agitation au réacteur IKA pendant 3h à 55°C, refroidissement dans un bain d'eau avec quelques glaçons pour éviter des écarts de température trop important, centrifugation 5 minutes à 6000t/min, séparation solide/liquide). A ce stade, la fraction liquide peut être congelée et lyophilisée et constitue les alginates semi-raffinés sous forme d'alginates de sodium. Afin d'obtenir des alginates raffinés, une purification est apportée aux étapes précédentes. Après séparation des résidus algaux, cette dernière étape de purification consiste en une précipitation de l'acide alginique par abaissement du pH, suivie de plusieurs lavages à l'eau acide afin d'éliminer les co-produits. Une augmentation du pH avec Na₂CO₃ permet de nouveau de solubiliser les alginates de sodium en limitant les sels, par rapport à l'utilisation de la soude. Enfin, une étape finale de congélation puis lyophilisation permet d'arriver au produit final. Afin d'obtenir les oligoalginates, saturés ou insaturés, la solution d'alginate est traitée par voie enzymatique ou acide afin d'abaisser le degré de polymérisation des alginates de 20 à 3.

### A) sans neutralisation préalable du milieur réactionnel avant la réaction d'aminolyse

### 1) Réaction de butanolyse et de glycosylation de Fischer

1 g d'alginate de sodium (110 200 g/mol, Degré de polymérisation = 630, extrait de *Sargassum vulgare*) dont le ratio M/G est 0,71 (5,71 mmol, 1 éq) a été mélangé avec 3 mL d'eau distillée. 80 mL (150 éq) de butanol ont été ajoutés à la solution d'alginate sous agitation avec 927 µl d'acide méthane sulfonique (14,27 mmol, 2,5 éq). Le milieu a été agité à la température de reflux du butanol (130-135°C) pendant 24 heures. Les eaux présentes dans le milieu et celles formées au cours de la réaction ont été éliminées dans un Dean Stark rempli de butanol, par une distillation azéotropique eau-butanol. Etant plus dense que le butanol, l'eau se déplace vers le fond du Dean Stark et quelques ml de butanol passent dans le ballon pour conserver le volume initial. Après 24 heures, une chromatographie sur couche mince (CH₂Cl₂/CH₃OH 95/5 v/v) a été réalisée pour le milieu réactionnel pour s'assurer que le produit attendu a bien été synthétisé.

### 2) Réaction d'aminolyse

La température du milieu a été abaissée jusqu'à 60°C avant d'ajouter 3 équivalents molaires (17,14 mmol) d'amine grasse (Pour n = 11, 3,18 grammes et pour n = 17, 4,62 grammes) nécessaire pour augmenter le pH à 8,5. Après 30 minutes d'agitation à 65°C et sous pression réduite de 150 mbar, le butanol a été évaporé en diminuant la pression de 150 mbar à 6 mbar sous une période de 1 heure. Le milieu a été laissé sous pression réduite de 6 mbar pendant 1 heure et demi pour s'assurer de l'évaporation des traces de butanol qui se formaient.

Le résidu obtenu a été repris dans l'éther diéthylique puis filtré sur fritté et rincé plusieurs fois avec l'éther diéthylique pour éliminer les sels et le sucre de départ non réagi. Le filtrat (contenant les guluronamides et mannuronamides d'alkyle) a été concentré sous vide pour éliminer l'éther diéthylique. Nous obtenons ainsi une huile brune foncée.

Pour n = 11, après chromatographie éventuelle de l'huile obtenue sur colonne de gel de silice (80 grammes, en utilisant l'éluant CH₂Cl₂/CH₃OH 95/5 v/v), a été déterminée la présence de 0,91 g (2,18 mmol, 39% de rendement) d'un mélange de 6 formes d'isomères (C₂₂H₄₃NO₆, masse molaire = 417,59 g/mol) de *N*-(12-dodécyl)-*n*-butyl β-L-gulurofuranosiduronamide (26%), *N*-(12-dodécyl)-*n*-butyl α-L-gulurofuranosiduronamide (15%), *N*-(12-dodécyl)-*n*-butyl α-L-guluropyranosiduronamide (16%), *N*-(12-dodécyl)-*n*-butyl β-L-guluropyranosiduronamide (12%), *N*-(12-dodécyl)-*n*-butyl α-D-mannofuranosiduronamide (18%) et *N*-(12-dodécyl)-*n*-butyl α-D-mannopyranosiduronamide (14%). **Cette composition tensioactive a été nommée AlgN12.**

D'après l'analyse des spectres RMN 1D et 2D du brut réactionnel (avant purification), ont pu être calculés : le ratio pyranose/furanose des formes guluronamides (0,72), le ratio pyranose/furanose des formes mannuronamides (0,81) et le ratio mannuronamide/guluronamide (0,47).

En outre, la quantité des sels de mésylates de dodécylammonium formés au cours de la réaction d'aminolyse a pu être quantifiée. En partant de 1 g d'alginate (1 éq.), cette quantité formée a été de 1,8 équivalents molaires (0,010 moles, 2,89 grammes).

Caractérisation du sel d'amine associé: mésylate de dodécylammonium

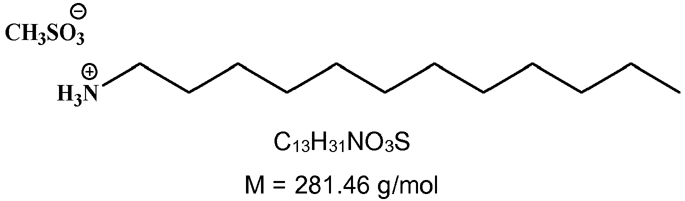

Pour n = 17, après chromatographie éventuelle de l'huile obtenuesur colonne de gel de silice (80 grammes, en utilisant l'éluant CH₂Cl₂/CH₃OH 95/5 v/v), a été déterminée la présence de1,03 g (2,05 mmol, 36% de rendement) d'un mélange de 6 formes d'isomères (C₂₈H₅₅NO₆, masse molaire = 501,75 g/mol) de *N*-(18-octadécyl)-*n*-butyl β-L-gulurofuranosiduronamide (25%), *N*-(18-octadécyl)-*n*-butyl α-L-gulurofuranosiduronamide (15%), *N*-(18-octadécyl)-*n*-butyl α-L-guluropyranosiduronamide (16%), *N*-(18-octadécyl)-*n*-butyl β-L-guluropyranosiduronamide (12%), *N*-(18-octadécyl)-*n*-butyl α-D-mannofuranosiduronamide (18%) et *N*-(18-octadécyl)-*n*-butyl α-D-mannopyranosiduronamide (14%). **Cette composition tensioactive a été nommée AlgN18.**

D'après l'analyse des spectres RMN 1D et 2D du brut réactionnel (avant purification), ont pu être calculés : le ratio pyranose/furanose des formes guluronamides (0,71), le ratio pyranose/furanose des formes mannuronamides (0,84) et le ratio mannuronamide/guluronamide (0,46). De même, la présence des sels de mésylates d'octadécylammonium formés lors de la réaction d'aminolyse a pu être quantifiée. En partant de 1 g d'alginate (1 éq.), cette quantité formée a été de 2 équivalents molaires (0,011 moles, 4,16 grammes).

Caractérisation du sel d'amine associé: mésylate d'octadécylammonium.

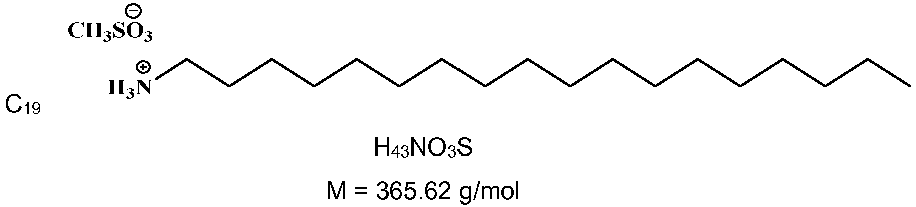

### B) avec neutralisation préalable du milieu réactionnel avant la réaction d'aminolyse

Dans le but de développer un procédé sans étape de purification derrière, plus simple à mettre en oeuvre à l'échelle pilote, tout en utilisant une quantité faible en amine et en évitant l'utilisation de solvants organiques (hormis le butanol), il a été procédé selon le protocole décrit ci-dessous :

### 1) Réaction de butanolyse et de glycosylation de Fischer

1 g d'alginate de sodium (110 200 g/mol, Degré de polymérisation = 630, extrait de *Sargassum vulgare*) dont le ratio M/G est 0,71 (5,71 mmol, 1 éq) a été mélangé avec 3 mL d'eau distillée. 80 mL (150 éq) de butanol ont été ajoutés à la solution d'alginate sous agitation avec 927 µl d'acide méthane sulfonique (14,27 mmol, 2,5 éq). Le milieu a été agité à la température de reflux du butanol (130-135°C) pendant 24 heures. Les eaux présentes dans le milieu et celles formées au cours de la réaction ont été éliminées dans un Dean Stark rempli de butanol, par une distillation azéotropique eau-butanol. Etant plus dense que le butanol, l'eau se déplace vers le fond du Dean Stark et quelques ml de butanol passe dans le ballon pour conserver le volume initial. Après 24 heures, une chromatographie sur couche mince (CH₂Cl₂/CH₃OH 95/5 v/v) a été réalisée pour le milieu réactionnel pour s'assurer que le produit attendu est bien synthétisé.

### 2) Neutralisation du milieu réactionnel

Afin de diminuer la quantité d'amines à ajouter lors de la seconde étape du procédé, le milieu réactionnel a été neutralisé, après refroidissement, avec de la soude 1 M (8 mL) jusqu'à un pH de 7. Ensuite, l'eau a été évaporée à l'évaporateur rotatif.

### 3) Réaction d'aminolyse

1 équivalent molaire de dodécylamine (5,71 mmol, 1,06 grammes) a été ajouté au milieu réactionnel. Après 30 minutes d'agitation à 65°C et sous pression réduite de 150 mbar, le butanol a été évaporé en diminuant la pression de 150 mbar à 6 mbar sous une période de 1 heure. Le milieu a été laissé sous pression réduite de 6 mbar pendant 1 heure et demi pour s'assurer de l'évaporation des traces de butanol qui se formaient.

Une étape pour éliminer les sels a consisté à ajouter 500 équivalents molaires de l'eau (60 mL) et l'ensemble a été agité à 70°C pendant 15 minutes.Après arrêt de l'agitation, les produits organiques amides floculaient à la surface de l'eau. En laissant le milieu refroidir à température ambiante, la phase organique s'est solidifiée, il a alors été facile d'éliminer l'eau chargé de sels, et les floculats solides ont été récupérés. Ces floculats, de coloration brune extrêmement intense, sont formés du brut de L-guluronamide et D-mannuronamide d'alkyle et des sels de mésylates de dodécylammonium. La masse finale obtenue en brut a été de 1,21 grammes (2,9 mmol). **Cette composition tensioactive a été nommée AlgN12brut.**

D'après l'analyse des spectres RMN 1D et 2D du brut réactionnel (sans purification), a été déterminée la présence d'un mélange de 6 formes d'isomères (C₂₂H₄₃NO₆, masse molaire = 417,59 g/mol) de *N*-(12-dodécyl)-*n*-butyl β-L-guiurofuranosiduronamide (24%), *N*-(12-dodécyl)-*n*-butyl α-L-gulurofuranosiduronamide (14%), *N*-(12-dodécyl)-*n*-butyl α-L-guluropyranosiduronamide (18%), *N*-(12-dodécyl)-*n*-butyl β-L-guluropyranosiduronamide (10%), *N*-(12-dodécyl)-*n*-butyl α-D-mannofuranosiduronamide (19%) et *N*-(12-dodécyl)-*n*-butyl α-D-mannopyranosiduronamide (15%).

Ces données ont permis de calculer le ratio pyranose/furanose des formes guluronamides (0,73), le ratio pyranose/furanose des formes mannuronamides (0,79) ainsi que le ratio mannuronamide/guluronamide (0,51).

De plus, la présence des sels de mésylates de dodécylammonium formés lors de la réaction d'aminolyse a pu être quantifiée. En partant de 1 g d'alginate (1 éq.), cette quantité formée a été de 0,4 équivalents molaires (0,0023 moles, 0,64 grammes).

### EXEMPLE 3 : MESURES DES TENSIONS INTERFACIALES (CAS DU SYSTÈME HUILE TOURNESOL-EAU) DES COMPOSITIONS GN12, GN18, AlgN12, Algn18 et Algn12brut

Les propriétés interfaciales des compositions tensioactives GN12, GN18, AlgN12, AlgN18 et Algn12brut ont été évaluées via la mesure des tensions interfaciales huile-eau. Les tensioactifs ont été solubilisés dans l'huile de tournesol à des concentrations variant de 0,12 à 2,28 g/L. Afin de favoriser la solubilité des tensioactifs dans l'huile, les solutions ont été laissées dans un bain aux ultrasons pendant 10 minutes à 50°C.

Les mesures des tensions à l'interface entre l'huile et l'eau ont été effectuées à 25°C avec un tensiomètre à anneau (Krüss, modèle K 100C). L'anneau utilisé était en platine iridié calibré.

La tension interfaciale entre l'huile de tournesol (marque Carrefour) et l'eau à 25°C a varié entre 24,71 et 25,04 mN/m.

Pour chaque composition tensioactive, l'appareil a mesuré initialement la tension de surface de l'huile de tournesol contenant le tensioactif (liquide de faible densité), puis la tension de surface de l'eau (liquide de haute densité). Enfin, l'huile a été ajoutée délicatement sur l'eau, tout en évitant la formation de bulles, l'appareil a commencé à mesurer la tension interfaciale entre l'huile de tournesol et l'eau (moyenne de 10 mesures).

**Tensions interfaciales des compositions tensioactives (mN/m)**

| | GN12 | AlgN12 | AlgN12 brut | GN18 | AlgN18 |
|---|---|---|---|---|---|
| 0,12 g/L | 18,91 | 18,45 | 17,25 | | |
| 0,25 g/L | 13,79 | 15,21 | 14,08 | 17,49 | 17,56 |
| 0,46 g/L | 12,45 | 11,75 | 11,29 | 14,315 | 14,37 |
| 0,61 g/L | 9,15 | 10,39 | 9,87 | | 13,02 |
| 1,11 g/L | 7,24 | 6,94 | | 10,9 | 9,63 |
| 1,64 g/L | 5,97 | | | 8,475 | |
| 2,28 g/L | | | | 6,75 | 6,22 |
| 2,97 g/L | | | | 5,83 | |
| 3,49 g/L | | | | 5,346 | |

| | | | | | |
|---|---|---|---|---|---|
| *Les cases grisées correspondent à des cas où la tension interfaciale n'a pas été mesurée* | | | | | |

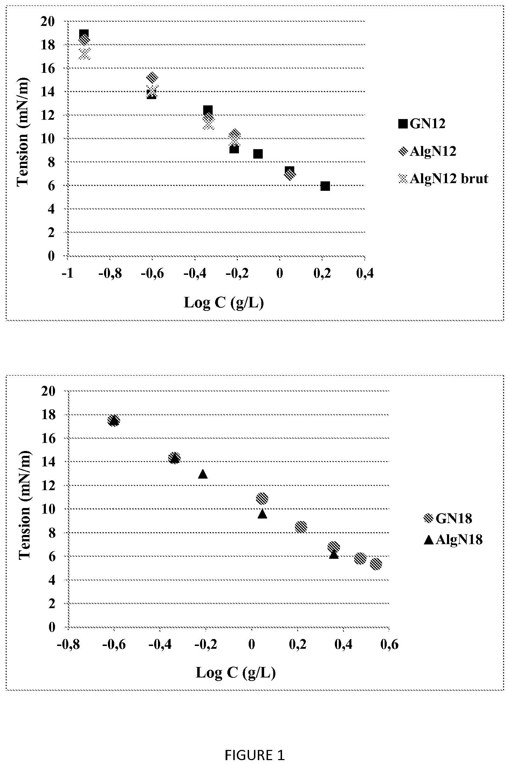

La figure 1 montre que les compositions tensioactives ont été capables de diminuer de façon similaire les tensions interfaciales eau/huile à des valeurs suffisamment faibles pour conférer aux compositions des pouvoirs émulsionnants. Il a été constaté que la composition AlgN12 brut obtenue par le procédé direct d'obtention des L-guluronamides et D-mannuronamides d'alkyle à partir de l'alginate, avec neutralisation préalable du milieu avant la réaction d'aminolyse était plus efficace que la composition tensioactive AlgN12 correspondant au mélange de tensioactifs L-guluronamides et D-mannuronamides d'alkyle purifiés par chromatographie (procédé direct d'obtention des L-guluronamides et D-mannuronamides d'alkyle à partir de l'alginate, sans neutralisation préalable du milieu avant la réaction d'aminolyse). Ces résultats ont montré la possibilité d'utiliser un brut de réaction sans nécessiter de purification supplémentaire. De même, les compositions dérivées de la dodécylamine (GN12, AlgN12, AlgN12brut) ont été plus efficaces que celles dérivées de l'octadécylamine (GN18, AlgN18).

### EXEMPLE 4 : MESURE DU POUVOIR ÉMULSIFIANT DES COMPOSITIONS GN12, GN18, AlgN12, Algn18 et Algn12brut

La stabilité des émulsions huile dans eau (H/E) et eau dans huile (E/H) formées à partir des compositions tensioactives GN12, GN18, AlgN12, AlgN18 et Algn12 brut a été étudiée en comparaison avec celle d'alkylpolyglycosides commerciaux : Montanov 82® de Seppic et Xyliance® de Soliance/ARD.

La stabilité des deux types émulsions H/E et E/H a été évaluée en considérant les deux ratios eau/huile 75/25 et 25/75 respectivement dans des tubes gradués à fond rond, 0,5% du produit tensioactif est introduit (20 mg). L'huile de tournesol a été introduite (1 ou 3 mL), puis les tensioactifs ont été solubilisés dans un bain aux ultrasons pendant 10 minutes à 50°C. Après la solubilisation de l'émulsifiant, l'eau ultrapure a été ajoutée (1 ou 3 mL).

Les deux phases ont ensuite été émulsionnées à l'aide d'un homogénéiseur Ultraturraxlka T18 basic® pendant 10 minutes à 11000 tours/min. L'emulsion a été placée dans un bain thermostaté à 20°C.

L'évolution de l'émulsion et sa démixtion progressive a été observée pendant quelques heures à plusieurs semaines.

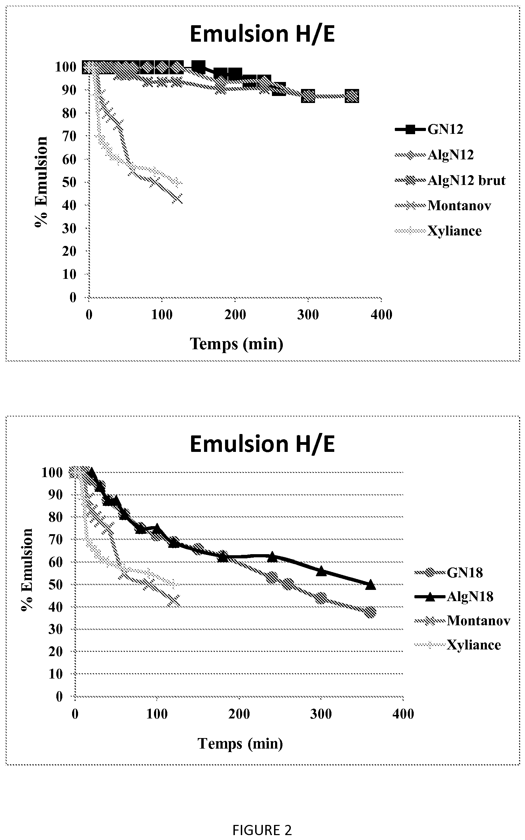

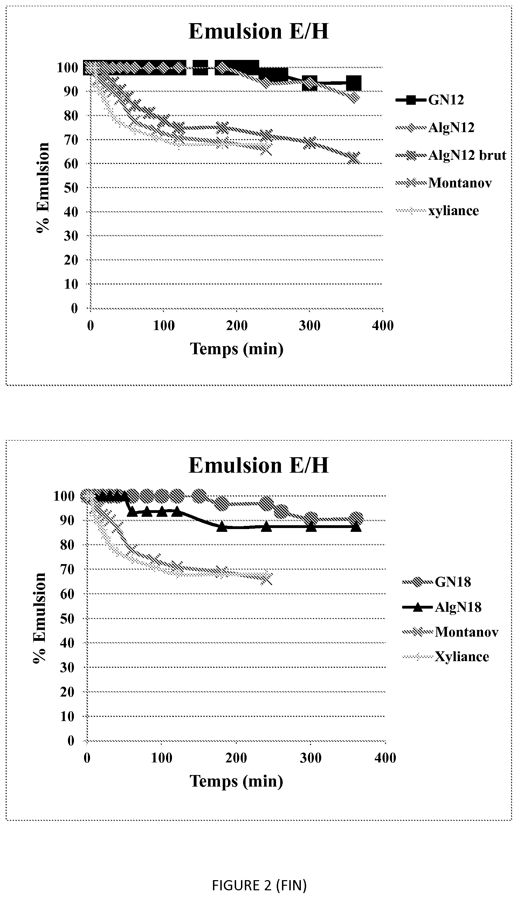

La figure 2 montre les résultats d'analyse du pouvoir émulsionnant des compositions de l'invention.

Les compositions tensioactives dérivées de la dodécylamine (GN12, AlgN12 et AlgN12brut) ont formé des émulsions H/E très stables, y compris dans le cas de la composition tensioactive brute (AlgN12 brut) ainsi que des émulsions E/H stables (GN12 et AlgN12). Les compositions tensioactives dérivées de l'octadécylamine (GN18 et AlgN18) ont formé des émulsions E/H très stables, les autres émulsions H/E ont subi une démixtion totale au bout de 5 h.

Pour les 2 types d'émulsions (E/H et H/E), les nouvelles compositions (GN12, AlgN12 et AlgN12brut) ont formé des émulsions plus stables que les références commerciales (Xyliance® et Montanov®).

### EXEMPLE 5 : ACTIVITÉ ANTIBACTÉRIENNE DE DIFFÉRENTES FRACTIONS DE PRODUITS MODIFIÉS

Deux protocoles ont été utilisés. Le premier (protocole A) a été appliqué à des fractions enrichies en isomères du *N*-(12-dodécyl)-*n*-butyl α-L-guluronamide par chromatographie sur gel de silice. Le second (Protocole B) a été suivi pour tester l'activité des compositions tensioactives dérivées de la dodécylamine (GN12, AlgN12 et AlgN12brut) et de la composition tensioactive dérivée de l'octadécylamine (AlgN18)

### Protocole A

### 1) Préparation du milieu de culture :

Le milieu de culture utilisé a été un mélange de 21 g/L Muller Hinton Broth et de 10 g/L d'agar dans l'eau. Ce mélange a été agité puis laissé bouillir. Ensuite, une étape d'autoclavage de ce mélange, pendant 30 minutes, a été nécessaire afin de le stériliser avant toute manipulation. Ce milieu de culture a été versé, à chaud, dans des boîtes de Petri, puis laissé refroidir.

### 2) Préparation des produits modifiés à tester :

5 mg de chaque sucre modifié ont été dissous dans 1 mL de DMSO. Une série de dilution à ½, par le DMSO, a ensuite été réalisée à partir de la solution mère, afin d'obtenir les concentrations 2,5 g/L, 1,25 g/L, 0,625 g/L et 0,3125 g/L.

### 3) Préparation des suspensions bactériennes et fongiques :

Les souches bactériennes utilisées ont été *Pseudomonas aeruginosa, Escherichia Coli, Enterococcus faecium* et *Staphylococcus aureus,* en plus de la souche fongique *Candida albicans.* 10⁶ bactéries ont été prélevées puis transférées dans une solution de NaCl 0,9%. Chaque boite de Petri, contenant le milieu Muller Hinton, a été inondée par une suspension bactérienne différente.

### 4) Protocole :

Après avoir laissé sécher les suspensions bactériennes sur la gélose, 10 µL de chaque solution à tester, et à différentes concentrations, ont été déposés sur la surface de la gélose inondée par la suspension bactérienne. Dans chaque boîte de Petri, 10 µL de DMSO ont été déposés comme contrôle négatif.

Les contrôles positifs utilisés ont été des disques imbibés d'ampicilline pour *Escherichia coli* et *Enterococcus faecium,* des disques de ceftazidime pour *Pseudomonas aeruginosa* et des disques de vancomycine pour *Staphylococcus aureus.*

Après séchage, les boîtes de Petri ont finalement été incubées à 37°C dans l'étuve, pendant 24 heures. L'activité antibactérienne a été évaluée par la mesure de la zone de clarification en mm tout autour du lieu de dépôt des différentes solutions à tester.

A titre d'exemple, l'activité des fractions I, Il et III enrichies en isomères du *N*-(12-dodécyl)-n-butyl α-L-guluronamide (forme β-L-furanoside = **Fraction I ;** forme α-L-pyranoside = **Fraction II** ; β-L-pyranoside = **Fraction III**) :

| Fractions enrichiesen | Concentration | *P*. *aeruginosa* | *E. coli* | *E. faecium* | *C albicans* | *S*. *aureus* |
|---|---|---|---|---|---|---|
| *N*-(12-dodécyl)-n-butyl-β-L-gulurofuranosiduronamide (75%) | 5 | 0 | 0 | 9 mm | 4,5 mm | 10,5 mm |
| | 2,5 | | | 9 mm | 4mm | 5mm |
| | 1,25 | | | 7mm | 4mm | 5mm |
| | 0,625 | | | 0 | 4mm | 0 |
| **Fraction I** | 0,3125 | | | 0 | 0 | 0 |
| *N*-(12-dodécyl)-n-butyl-α-L-guluropyranosiduronamide (74%) | 5 | 0 | 0 | 20 mm | 5 mm | 18,5 mm |
| | 2,5 | | | 20 mm | 3 mm | 17 mm |
| | 1,25 | | | 11 mm | 3 mm | 5 mm |
| | 0,625 | | | 8 mm | 0 | 0 |
| **Fraction II** | 0,3125 | | | 8 mm | 0 | 0 |
| *N*-(12-dodécyl)-n-butyl-β-L-guluropyranosiduronamide (94%) | 5 | 0 | 0 | 0 | 9 mm | 20,5 mm |
| | 2,5 | | | | 7 mm | 18 mm |
| | 1,25 | | | | 5 mm | 0 |
| | 0,625 | | | | 0 | 0 |
| **Fraction III** | 0,3125 | | | | 0 | 0 |
| Contrôle positif | - | Ceftazidime 28 mm | Ampicilline 22 mm | Ampicilline 26 mm | - | Vancomycine 19 mm |

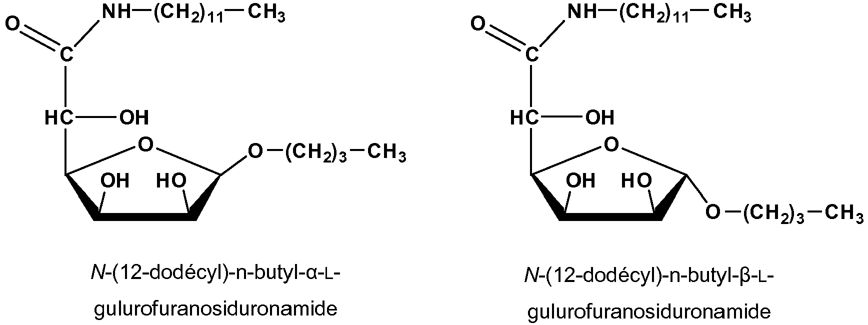

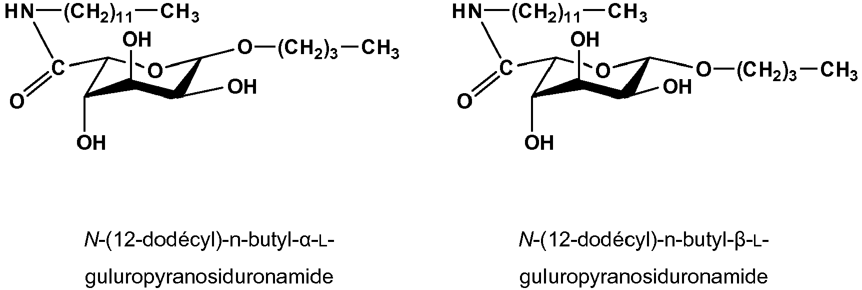

| Fractions enrichiesen | Concentration | *P*. *aeruginosa* | *E. coli* | *E. faecium* | *C albicans* | *S*. *aureus* |
|---|---|---|---|---|---|---|
| *N*-(18-octadécyl)-n-butyl-α-L-gulurofuran osiduronamide (47%) | 5 | 0 | 0 | 6 mm | 0 | 0 |
| | 2,5 | | | 6 mm | | |
| | 1,25 | | | 6 mm | | |
| | 0,625 | | | 4mm | | |
| | 0,3125 | | | 0 | | |
| *N*-(18-octadécyl)-n-butyl-β-L-gulurofuranosiduronamide (63%) | 5 | 0 | 0 | 4,5 mm | 0 | 10 mm |
| | 2,5 | | | 4mm | | 0 |
| | 1,25 | | | 4mm | | 0 |
| | 0,625 | | | 3 mm | | 0 |
| | 0,3125 | | | 0 | | 0 |
| *N*-(18-octadécyl)-n-butyl-β-L-guluropyranosiduronamide (86%) | 5 | 0 | 0 | 6 mm | 0 | 0 |
| | 2,5 | | | 4mm | | |
| | 1,25 | | | 0 | | |
| | 0,625 | | | 0 | | |
| | 0,3125 | | | 0 | | |
| Contrôle positif | - | Ceftazidime 28 mm | Ampicilline 22 mm | Ampicilline 26 mm | - | Vancomycine 19 mm |

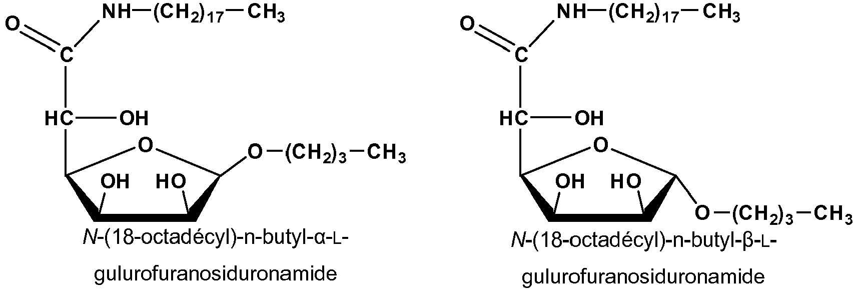

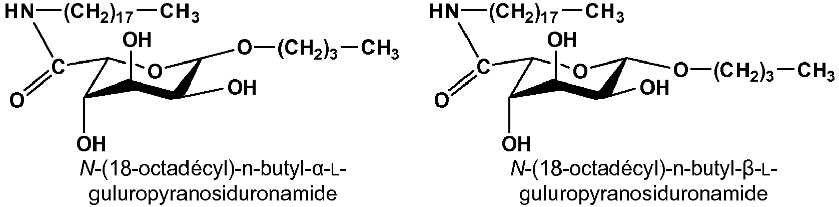

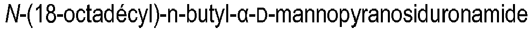

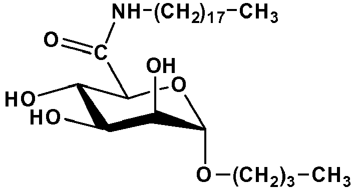

Pour les études des activités antibactériennes et antifongiques, les fractions issues de la purification du brut *N*-(12-dodécyl)-*n*-butyl-L-guluronamide ont été testées. Chaque fraction est plus enrichie en un isomère que les autres.

Contre les bactéries gram positif *Enterococcus faecium* et *Staphylococcus aureus,* les *N*-(12 dodécyl)-*n*-butyl L-guluronamide (fraction enrichie en forme pyranose α) ont montré une capacité importante à inhiber la croissance de ces deux bactéries de l'ordre de 20 mm et 18,5 mm, respectivement. Cette activité inhibitrice a diminué en diminuant la concentration. Il semble que les formes pyranoses, présentes majoritairement (94%), n'aient montré aucun effet inhibiteur contre *Enterococcus faecium,* mais aient montré un effet plus important sur la croissance de la levure *Candida albicans* (9 mm à 5 g/L), en plus de l'effet inhibiteur le plus fort contre *Staphylococcus aureus* (20,5 mm à 5 g/L).

Par contre, les *N*-(18 octadécyl)-*n*-butyl L-guluronamide n'ont montré aucun effet inhibiteur ni sur la bactérie gram positif *Staphylococcus aureus,* ni sur la levure *Candida albicans.*

Contre les bactéries gram négatif *Pseudomonas aeruginosa* et *Escherichia coli,* ni les *N*-(12 dodécyl)-n-butyl L-guluronamide et ni les *N*-(18 octadécyl)-*n*-butyl L-guluronamide (que ce soit sous forme furanose ou pyranose) n'ont montré une activité inhibitrice de la croissance de ces deux bactéries.

### Protocole B

Les résultats intéressants de l'activité antibactérienne et antifongique, obtenus pour les fractions enrichies en isomères du *N*-(12-dodécyl)-*n*-butyl α-L-guluronamide, ont encouragé les inventeurs à tester l'activité antibactérienne et antifongique des mélanges des produits tensioactifs obtenus à partir des guluronates (GN12) et à partir des alginates (AlgN12, AlgN12 brut et AlgN18). Pour ces produits mélanges de tensioactifs, une nouvelle méthode à été testée basée sur l'étude de la capacité des tensioactifs de l'invention à tuer les bactéries et puis le comptage du nombre de bactéries vivantes sur la gélose de Muller-Hinton.

### 1) Préparation de l'inoculum bactérien et fongique :

L'inoculum a été préparé à une turbidité équivalente à 0,5 MacFarland (Biomérieux France), puis dilué à 1/100 (10⁶ UFC/ml). De cet inoculum, une série des dilutions 10⁻¹,10⁻²,10⁻³,10⁻⁴,10⁻⁵,10⁻⁶ a été effectuée. 100 µl de chaque dilution ont été étalés (méthode de numération) à la surface d'une gélose de Muller-Hinton (détermination de nombre des bactéries en UFC/ml dans l'inoculum 'N'.

### 2) Préparation des produits modifiés à tester :

Des solutions mères ont été préparées pour chaque tensioactif GN12 (430 mg/ml), AlgN12 (310 mg/ml), AlgN12 brut (216 mg/ml) et AlgN18 (219 mg/ml). Une série de dilutions à raison ½, par le DMSO a étéréalisée pour chaque produit dans un bouillon de Muller-Hinton, la dilution finale était 1/128.

### 3) Protocole :

Dans chaque tube des dilutions de tensioactif, 1 ml de l'inoculum bactérien a été ajouté.

Après incubation de 24h à 36°C, 100 µl de chaque tube clair ont été étalés à la surface d'une gélose Muller-Hinton suivi par une incubation 24h à 37°C.

Le nombre des bactéries vivantes a été déterminé : N0=nx10 UFC/ml (n=nombre des colonies).

Le pourcentage des bactéries vivantes a été calculé : N0/N x100 :

| **Nom de la bactérie** | **Nom du produit** | **Concentration préparée (mg/ml)** | **Résultats** |
|---|---|---|---|
| *Escherichia coli* | AlgN₁₂ | | 155 mg/ml => Inhibition de 100% des bactéries |
| | | | 77,5 mg/ml => Inhibition de 100% des bactéries |
| | | 310 mg/ml | 38,75 mg/ml => Inhibition de 100% des bactéries |
| | | | 19,375 mg/ml => Inhibition de 99,99% des bactéries |
| | AlgN₁₂ (Brut) | 216 mg/ml | 108 mg/ml => Inhibition de 100% des bactéries |
| | | | 54 mg/ml => Inhibition de 100% des bactéries |
| | | | 27 mg/ml => Inhibition de 100% des bactéries |
| | | | 13,5 mg/ml => Inhibition de 99,99% des bactéries |
| | AlgN₁₈ | 219 mg/ml | 109,5 mg/ml => Inhibition de 100% des bactéries |
| | | | 54,75 mg/ml => Inhibition de 100% des bactéries |
| | | | 27,375 mg/ml => Inhibition de 99,99% des bactéries |
| | GN₁₂ | 430 mg/ml | 215 mg/ml => Inhibition de 100% des bactéries |
| | | | 107,5 mg/ml => Inhibition de 99,99% des bactéries |
| | AlgN₁₂ | 310 mg/ml | 155 mg/ml => Inhibition de 100% des bactéries |
| | | | 77,5 mg/ml => Inhibition de 100% des bactéries |
| | | | 38,75 mg/ml => Inhibition de 100% des bactéries |
| | | | 19,375 mg/ml => Inhibition de 99,9% des bactéries |
| | AlgN₁₂ (Brut) | 216 mg/ml | 108 mg/ml => Inhibition de 100% des bactéries |
| *Pseudomonas aeruginosa* | | | 54 mg/ml => Inhibition de 100% des bactéries |
| | | | 27 mg/ml => Inhibition de 100% des bactéries |
| | | | 13,5 mg/ml => Inhibition de 99,9% des bactéries |
| | AlgN₁₈ | 219 mg/ml | 109,5 mg/ml => Inhibition de 100% des bactéries |
| | | | 54,75 mg/ml => Inhibition de 100% des bactéries |
| | | | 27,375 mg/ml => Inhibition de 100% des bactéries |
| | GN₁₂ | 430 mg/ml | 215 mg/ml => Inhibition de 100% des bactéries |
| | | | 107,5 mg/ml => Inhibition de 100% des bactéries |
| | | | 53,75 mg/ml => Inhibition de 100% des bactéries |
| *Enterococcus faecium* | AlgN₁₂ | 310 mg/ml | 2,42 mg/ml => Inhibition de 100% des bactéries |
| | AlgN₁₂ (Brut) | 216 mg/ml | 1,7 mg/ml => Inhibition de 100% des bactéries |
| | AlgN₁₈ | 219 mg/ml | 109,5 mg/ml => Inhibition de 100% des bactéries |
| | | | 54,75 mg/ml => Inhibition de 100% des bactéries |
| | | | 27,375 mg/ml => Inhibition de 100% des bactéries |
| | G N₁₂ | 430 mg/ml | 3,36 mg/ml => Inhibition de 100% des bactéries |
| *Candidas albicans* | AlgN₁₂ | 310 mg/ml | 2,42 mg/ml => Inhibition de 100% des bactéries |
| | AlgN₁₂ (Brut) | 216 mg/ml | 1,7 mg/ml => Inhibition de 100% des bactéries |
| | AlgN₁₈ | 219 mg/ml | 109,5 mg/ml => Inhibition de 100% des bactéries |
| | | | 54,75 mg/ml => Inhibition de 100% des bactéries |
| | | | 27,375 mg/ml => Inhibition de 100% des bactéries |
| | G N₁₂ | 430 mg/ml | 3,36 mg/ml => Inhibition de 100% des bactéries |
| *Staphylococcus aureus* | AlgN₁₈ | 219 mg/ml | 109,5 mg/ml => Inhibition de 100% des bactéries |

Contre la bactérie gram positif *Enterococcus faecium,* le mélange d'AlgN12 brut (avec une neutralisation préalable à la réaction d'aminolyse) paraît plus efficace que le mélange AlgN12 (procédé sans neutralisation préalable à la réaction d'aminolyse) car une concentration de 1,7 mg/ml de AlgN12 (brut) suffira d'inhiber 100% d'*Enterococcus faecium* alors que cette concentration est plus élevée dans le cas d'AlgN12 (2,42 mg/ml). De plus, le mélange de tensioactifs issus de la modification chimique de l'alginate (mélange de mannuronamide et guluronamide) présente un pouvoir plus fort contre *Enterococcus faecium* que les tensioactifs à base de guluronamides seulement (concentration plus élevée en GN12 de l'ordre de 3,36 mg/ml pour inhiber 100% d'*Enterococcus faecium*). Cette observation est la même pour l'inhibition de *Candida albicans.*

Contre les bactéries gram négatif *Pseudomonas aeruginosa* et *Escherichia coli,* des concentrations très élevées en tensioactifs sont nécessaires pour inhiber à 100% ces deux bactéries ; montrant que AlgN12 (38,75 mg/ml), AlgN12 brut (13,5 mg/ml) et GN12 (53,75 mg/ml) ont un faible pouvoir antibactérien contre ces deux types de bactéries.

En comparant les AlgN12 et les AlgN18, le produit tensioactif amide d'alginate ayant une chaîne octadécyle présente un pouvoir antibactérien plus faible contre les bactéries gram positif et gram négatif testées. Une concentration élevée en AlgN18 est nécessaire pour inhiber 100% de bactéries *Enterococcus faecium* (27,375 mg/ml), *Pseudomonas aeruginosa* (27,375 mg/ml), *Escherichia coli* (54,75 mg/ml) et *Candida albicans* (27,375 mg/ml).

### Listes des références

1- Hill et Lehen-Ferrenbach, "In Sugar-based surfactants fundamentals and Applications", C.C. Ruiz (Ed.), 1-20, CRC Press, ISBN 978-1-4200-5166-7, 2009
2- Laurent et al., J. Surfact. Deterg., 14 : 51-63, 2011
3- Demande internationale WO 9206984
4- Demande internationale WO 9303004
5- Brevet US 7,655,611
6- Brevet US 5,872,111
7- Brevet US 2,670,345
8- Benvegnu et Sassi, Topics in Current Chemistry, 294 : 143-164, 2010
9- Demande internationale WO 03/104248
10- Demande internationale WO 03/099870
11- Demande internationale WO 2009/134368
12- Demande internationale WO 98/40511

## Revendications

1. Procédé de préparation d'une composition comprenant :
(i) des L-guluronamides d'alkyle de formules (la) et (Ib) : 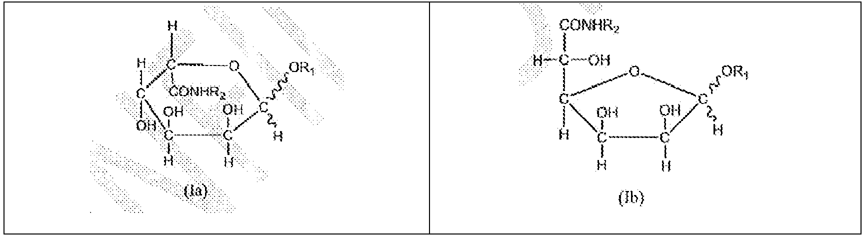 ou
(ii) un mélange de L-guluronamides d'alkyle de formules (la) et (Ib) et de D-mannuronamides d'alkyle de formule (IIa) et (IIb) : 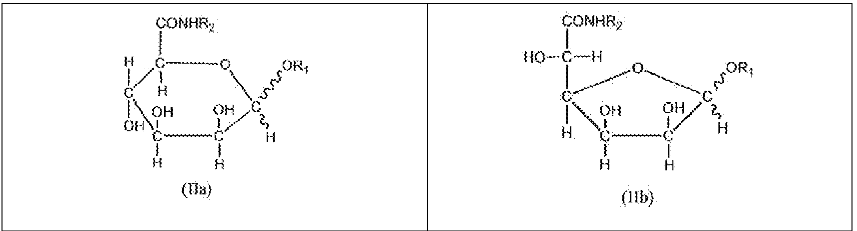 où
- R₁ est une chaîne alkyle de 2 à 22 atomes de carbone, linéaire ou ramifiée, saturée ou insaturée ;
- R₂ est un atome d'hydrogène, une chaîne alkyle de 2 à 22 atomes de carbone linéaire ou ramifiée, saturée ou insaturée pouvant comporter une fonction amine terminale ;
et **caractérisé en ce que** ledit procédé comprend :
a) une étape de réaction de butanolyse et de glycosylation de Fischer à partir de poly(oligo)guluronates, d'oligoalginates, d'alginates et/ou d'algues brunes ;
b) une étape de réaction d'aminolyse sur le milieu réactionnel issu de l'étape a), en présence d'une amine de formule R'NH₂, linéaire ou ramifiée, saturée ou insaturée où R' est composé de 2 à 22 atomes de carbone.

2. Procédé selon la revendication 1, où ledit procédé comprend une étape a') de neutralisation du milieu réactionnel issu de l'étape a) avant l'étape b).

3. Procédé selon la revendication 1 ou 2, où l'étape a) est réalisée en présence (i) d'eau et/ou d'un solvant ionique et/ou d'un solvant eutectique, (ii) d'un alcool de formule ROH, linéaire ou ramifié, saturé ou insaturé, ayant de 1 à 4 atomes de carbone, et (iii) d'un catalyseur acide.

4. Procédé selon la revendication 3, où le catalyseur acide est choisi dans le groupe constitué de : l'acide chlorhydrique, l'acide sulfurique, un acide alkyl sulfurique, un acide sulfonique, un acide alkylsulfonique ou un sulfosuccinate d'alkyl, les acides perhalohydriques, des métaux, leurs oxydes ou leurs sels comme leurs halogénures.

5. Procédé selon la revendication 4, où le catalyseur acide est l'acide méthanesulfonique.

6. Procédé selon l'une quelconque des revendications 1 à 4, où l'alcool ROH est le n-butanol.

7. Procédé selon l'une quelconque des revendications 1 à 6, où l'étape b) est réalisée en présence d'une amine grasse choisie dans le groupe constitué de dodécylamine et de l'amine oléïque.

8. Composition obtenue par un procédé selon l'une quelconque des revendications 1 à 7.

9. Composition selon la revendication 8, où ladite composition est une émulsion huile dans eau ou eau dans huile.

10. Utilisation d'une composition selon la revendication 8 ou 9 comme agent tensioactif.

11. Utilisation d'une composition selon la revendication 10, où l'agent tensioactif est choisi dans le groupe constitué des agents solubilisants, hydrotropes, mouillants, moussants, émulsionnants, émulsifiants et/ou détergents.

12. Utilisation non-thérapeutique d'une composition la revendication 8 ou 9 comme agent antibactérien et/ou antifongique.

13. Tensioactif comprenant une composition selon la revendication 8 ou 9.

14. Antibactérien et/ou antifongique comprenant une composition selon la revendication 8 ou 9.

## Patentansprüche

1. Verfahren zur Herstellung eines Zusammensetzung, die Folgendes umfasst:
(i) Alkyl-L-guluronamide nach den Formeln (Ia) und (Ib) : 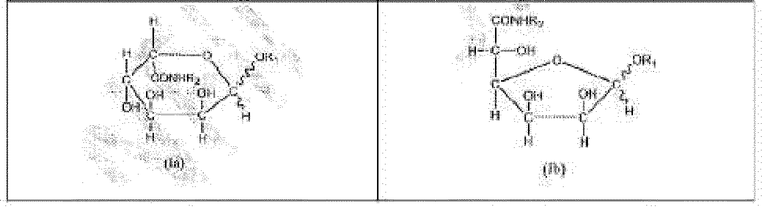 oder
(ii) eine Mischung aus Alkyl-L-guluronamiden nach den Formeln (Ia) und (Ib) und aus Alkyl-D-mannuronamiden nach den Formeln (IIa) und (IIb) : 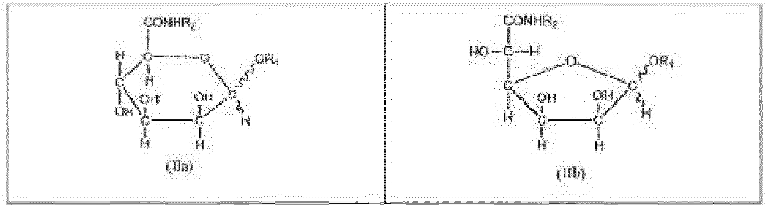 wobei
- R₁ für eine Alkylkette mit 2 bis 22 Kohlenstoffatomen steht, die geradkettig oder verzweigt, gesättigt oder ungesättigt ist;
- R₂ für ein Wasserstoffatom, für eine Alkylkette mit 2 bis 22 Kohlenstoffatomen steht, die geradkettig oder verzweigt, gesättigt oder ungesättigt ist, wobei sie eine endständige Aminfunktion aufweisen kann;
und **dadurch gekennzeichnet, dass** das Verfahren Folgendes umfasst:
a) einen Schritt der Butanolysereaktion und der Fischer-Glykosylierung ausgehend von Poly(oligo)guluronaten, Oligoalginaten, Alginaten und/oder Braunalgen;
b) einen Schritt der Aminolysereaktion, welcher an dem Reaktionsmilieu vorgenommen wird, das aus dem Schritt a) stammt, in Gegenwart eines Amins mit der Formel R'NH2, welches geradkettig oder verzweigt, gesättigt oder ungesättigt ist, wobei R' sich aus 2 bis 22 Kohlenstoffatomen zusammensetzt.

2. Verfahren nach Anspruch 1, wobei das Verfahren einen Schritt a') umfasst, in welchem vor dem Schritt b) das Reaktionsmilieu neutralisiert wird, das im Schritt a) erhalten wurde.

3. Verfahren nach Anspruch 1 oder 2, wobei der Schritt a) in Gegenwart (i) von Wasser und/oder eines ionischen Lösungsmittels und/oder eines eutektischen Lösungsmittels, (ii) eines Alkohols mit der Formel ROH, welcher geradkettig oder verzweigt, gesättigt oder ungesättigt ist, wobei er 1 bis 4 Kohlenstoffatome hat, und (iii) eines sauren Katalysators durchgeführt wird.

4. Verfahren nach Anspruch 3, wobei der saure Katalysator aus der Gruppe ausgewählt ist, die aus Folgendem besteht: Salzsäure, Schwefelsäure, einer Alkylschwefelsäure, einer Sulfonsäure, einer Alkylsulfonsäure oder einem Alkylsulfosuccinat, den Perhalogenwasserstoffsäuren, Metallen, deren Oxiden oder deren Salzen wie ihren Halogeniden.

5. Verfahren nach Anspruch 4, wobei es sich bei dem sauren Katalysator um Methansulfonsäure handelt.

6. Verfahren nach einem beliebigen der Ansprüche 1 bis 4, wobei es sich bei dem Alkohol ROH um n-Butanol handelt.

7. Verfahren nach einem beliebigen der Ansprüche 1 bis 6, wobei der Schritt b) in Gegenwart eines Fettamins durchgeführt wird, das aus der Gruppe ausgewählt ist, welche aus Dodecylamin und Oleylamin besteht.

8. Zusammensetzung, die mittels eines Verfahrens nach einem beliebigen der Ansprüche 1 bis 7 erhalten wurde.

9. Zusammensetzung nach Anspruch 8, wobei es sich bei der Zusammensetzung um eine Öl-in-Wasser- oder Wasser-in-Öl-Emulsion handelt.

10. Verwendung einer Zusammensetzung nach Anspruch 8 oder 9 als Tensid.

11. Verwendung einer Zusammensetzung nach Anspruch 10, wobei das Tensid aus der Gruppe ausgewählt ist, die aus Lösungsvermittlern, hydrotropen Stoffen, Netzmitteln, Schaumbildnern, Emulsionsbildner, Emulgatoren und/oder Detergenzien besteht.

12. Nicht-therapeutische Verwendung einer Zusammensetzung nach Anspruch 8 oder 9 als antibakterielles und/oder pilzbekämpfendes Mittel.

13. Tensid, das eine Zusammensetzung nach Anspruch 8 oder 9 umfasst.

14. Antibakterielles und/oder pilzbekämpfendes Mittel, das eine Zusammensetzung nach Anspruch 8 oder 9 umfasst.

## Claims

1. Method for preparing a composition comprising:
(i) alkyl L-guluronamides of formulae (Ia) and (Ib): 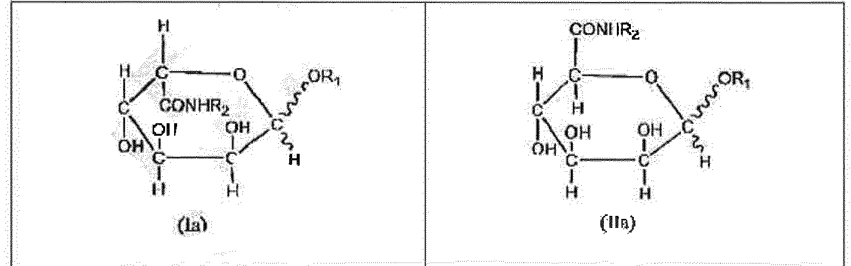 or
(ii) a mixture of alkyl L-guluronamides of formulae (Ia) and (Ib) and of alkyl D-mannuronamides of formulae (IIa) and (IIb): 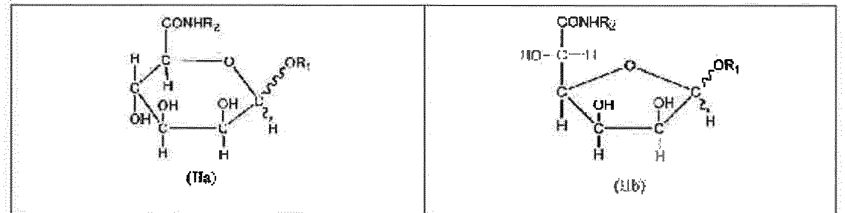 in which:
- R₁ is a linear or branched, saturated or unsaturated alkyl chain of 2 to 22 carbon atoms;
- R₂ is a hydrogen atom, a linear or branched, saturated or unsaturated alkyl chain of 2 to 22 carbon atoms which may comprise a terminal amine function;
and **characterized in that** said process comprises:
a) a butanolysis and Fischer glycosylation reaction step using poly(oligo)guluronates, oligoalginates, alginates and/or brown algae;
b) a step of aminolysis reaction on the reaction medium derived from step a), in the presence of a linear or branched, saturated or unsaturated amine of formula R'NH₂ in which R' is composed of 2 to 22 carbon atoms.

2. Method as claimed in claim 1, in which said process comprises a step a') of neutralizing the reaction medium derived from step a) before step b).

3. Method as claimed in claim 1 or 2, in which step a) is performed in the presence (i) of water and/or of an ionic solvent and/or of a eutectic solvent, (ii) of a linear or branched, saturated or unsaturated alcohol of formula ROH, containing from 1 to 4 carbon atoms, and (iii) of an acid catalyst.

4. Method as claimed in claim 3, in which the acid catalyst is chosen from the group constituted by: hydrochloric acid, sulfuric acid, an alkylsulfuric acid, a sulfonic acid, an alkylsulfonic acid or an alkyl sulfosuccinate, perhalohydric acids, metals, oxides thereof or salts thereof such as the halides thereof.

5. Method as claimed in claim 4, in which the acid catalyst is methanesulfonic acid.

6. Method as claimed in any one of claims 1 to 4, in which the alcohol ROH is *n*-butanol.

7. Method as claimed in any one of claims 1 to 6, in which step b) is performed in the presence of a fatty amine chosen from the group constituted by dodecylamine and oleylamine.

8. Composition obtained via a process as claimed in any one of claims 1 to 7.

9. Composition as claimed in claim 8, in which said composition is an oil-in-water or water-in-oil emulsion.

10. Use of a composition as claimed in claim 8 or 9 as a surfactant.

11. Use of a composition as claimed in claim 10, in which the surfactant is chosen from the group constituted by solubilizers, hydrotropes, wetting agents, foaming agents, emulsifying agents, emulsifiers and/or detergents.

12. Non-therapeutic use of a composition as claimed in claim 8 or 9 as an antibacterial and/or antifungal agent.

13. Surfactant comprising a composition as claimed in claim 8 or 9.

14. Antibacterial and/or antifungal comprising a composition as claimed in claim 8 or 9.
